**Europäisches Patentamt**

**European Patent Office**

**Office européen des brevets**

(19)

(11) Numéro de publication : **0 011 042**
**B2**

(12) # NOUVEAU FASCICULE DE BREVET EUROPÉEN

(45) Date de publication du nouveau fascicule du brevet :
26.03.86

(51) Int. Cl.⁴ : **C 07 C 47/06**, C 07 C 45/49

(21) Numéro de dépôt : **79420052.7**

(22) Date de dépôt : **22.10.79**

(54) **Procédé de préparation de l'acétaldéhyde.**

(30) Priorité : 31.10.78 FR 7831634

(43) Date de publication de la demande :
14.05.80 Bulletin 80/10

(45) Mention de la délivrance du brevet :
09.09.81 Bulletin 81/36

(45) Mention de la décision concernant l'opposition :
26.03.86 Bulletin 86/13

(84) Etats contractants désignés :
**AT BE CH DE FR GB IT LU NL SE**

(56) Documents cités :
**DE-A- 877 598**
**Bull. Chem. Soc. Jpn. 37 (1964), S. 236 bis 241**
**"Chemierohstoffe aus Kohle", G. Thieme Verlag**
**1977, S. 329 bis 333**
**Chemie-Ing.-Techn. 37 (1965), Seiten 383-388**
**Le dossier contient des informations techniques pré-**
**sentées postérieurement au dépôt de la demande et**
**ne figurant pas dans le présent fascicule.**

(73) Titulaire : **RHONE-POULENC CHIMIE DE BASE**
**25, quai Paul Doumer**
**F-92408 Courbevoie (FR)**

(72) Inventeur : **Gauthier-Lafaye, Jean**
**21, rue Duguesclin**
**F-69006 Lyon (FR)**
Inventeur : **Perron, Robert**
**La Pecolière**
**F-69390 Charly (FR)**

(74) Mandataire : **Varnière-Grange, Monique et al**
**RHONE-POULENC RECHERCHES Service Brevets**
**Chimie et Polymères Centre de Recherches de**
**Saint-Fons 85, avenue des Frères Perret B.P. 62**
**F-69192 St-Fons Cédex (FR)**

EP 0 011 042 B2

Jouve, 18, rue St-Denis, 75001 Paris, France

## Description

La présente invention a pour objet un procédé de préparation de l'acétaldéhyde par carbonylation du méthanol en présence d'hydrogène.

L'acétaldéhyde est un intermédiaire de grand intérêt dans l'industrie chimique. Il est utile, notamment, pour la fabrication de l'acide acétique et de l'anhydride acétique. (Cf. « Encyclopedia of Chemical Technology » KIRK-OTHMER. 3e Edition, Volume 1, pages 97 et suivantes).

Des procédés industriels ont été développés pour produire de l'acétaldéhyde. Le procédé le plus utilisé actuellement est l'oxydation directe de l'éthylène. Toutefois, cet hydrocarbure étant issu du pétrole, il peut devenir plus économique de choisir des matières premières issues du gaz de synthèse. telles que le méthanol.

L'utilisation du méthanol comme matière première dans la synthèse de produits, qui traditionnellement proviennent à l'échelle industrielle de l'éthylène, a constitué l'objet et continue d'être l'objet de nombreuses recherches. Ces recherches visent essentiellement l'application des techniques de carbonylation, c'est-à-dire de la réaction du monoxyde de carbone sur le méthanol, éventuellement en présence d'hydrogène.

C'est ainsi que de nombreux travaux antérieurs ont porté sur l'homologation du méthanol, c'est-à-dire sur la production d'éthanol par carbonylation du méthanol.

Il est bien connu que le méthanol réagit avec un mélange d'oxyde de carbone et d'hydrogène (1 : 1) à 185 °C et sous 360 atmosphères en présence de dicobaltoctacarbonyle. Dans ces conditions, on obtient un mélange de divers produits, renfermant de l'éthanol avec une sélectivité relativement faible (Cf. WENDER et Coll. « Science » volume 113, page 206, 1951).

D'autres auteurs ont montré que lorsque la réaction d'homologation est conduite à 200 °C sous 200 à 350 atmosphères en présence de diacétate de cobalt, le rendement en éthanol peut être amélioré en opérant simultanément en présence d'un promoteur iodé ($I_2$ ou $CH_3I$) et d'un mélange gazeux riche en oxyde de carbone. (Cf. BERTY et coll. « Chem. Tech. » volume 5, pages 260-266, 1956).

D'autres progrès vers la production d'éthanol ont pu être réalisés par adjonction au système catalytique précédent d'un composé phosphoré soluble dans le méthanol (Cf. brevet français n° 1 341 840), par introduction d'halogénures de ruthénium et d'osmium (Cf. brevet des Etats-Unis d'Amérique n° 3 285 948), ou bien par addition d'une phosphine tertiaire et d'un hydrocarbure comme solvant. (Cf. brevet français n° 2 314 166).

Néanmoins, ces procédés ne sont pas applicables à l'échelle industrielle : ils ne permettent pas d'atteindre des sélectivités élevées en éthanol et par suite nécessitent la mise en place d'installations complexes pour séparer les divers constituants du mélange obtenu ce qui alourdit de manière inacceptable l'économie globale d'un tel procédé.

Partant de cette constatation, d'autres auteurs se sont orientés vers la production d'acétaldéhyde. C'est ainsi qu'il a été mentionné que la présence d'une quantité de cobalt inférieure à 2 millimoles par mole de méthanol dans un système catalytique contenant un promoteur halogéné favorise la conversion du méthanol en acétaldéhyde (Cf. brevet des Etats-Unis d'Amérique n° 3 356 734).

Effectivement, lorsque l'on réalise la carbonylation du méthanol selon la technique objet du brevet américain précité, à 185 °C sous 300 à 400 atm avec un rapport molaire $CO/H_2$ de 1.4 pendant 2 heures, on obtient environ 130 g d'acétaldéhyde par litre de milieu réactionnel et par heure, pour une productivité de l'ordre de 70 g d'acétaldéhyde par heure et par gramme de cobalt engagé dans la réaction, compte tenu du fait que le diméthoxyéthane formé est une source potentielle de l'acétaldéhyde.

Des travaux plus récents (Cf. demandes de brevets japonais 77/136 111 et 77/133 914) ont montré que les résultats atteints à l'aide de ce système catalytique peuvent être sensiblement améliorés par adjonction au système (Cobalt, Iode) d'une quantité importante d'un composé phosphoré ou d'un composé de l'arsenic, de l'antimoine ou du bismuth. Néanmoins, la productivité en acétaldéhyde par rapport au cobalt engagé reste trop faible, pour que de tels procédés puissent présenter un intérêt industriel. En outre selon les procédés connus, une partie non négligeable du méthanol est convertie en butanol, butanal et buténal, produits dont la valorisation est aléatoire, et qui grèvent l'économie globale du procédé en nécessitant des étapes supplémentaires de séparation.

Il a maintenant été trouvé un procédé pour carbonyler le méthanol sélectivement en acétaldéhyde, procédé qui permet d'obvier aux inconvénients précités.

La présente invention a donc pour objet un procédé de carbonylation du méthanol en présence d'hydrogène et de cobalt, qui permet notamment d'augmenter considérablement la productivité horaire en acétaldéhyde par rapport à la quantité de cobalt engagée dans la réaction, et cela, pratiquement sans perte de productivité par rapport au volume réactionnel.

Les recherches, qui ont abouti à la présente invention, ont montré d'une manière tout à fait inattendue que l'on peut obtenir sélectivement l'acétaldéhyde, éventuellement sous la forme du diméthylacétal correspondant, en faisant réagir sur le méthanol un mélange comprenant de l'hydrogène et du monoxyde de carbone, l'hydrogène représentant au moins 25 % en mole, à une température de l'ordre de 165 °C au moins, en présence d'une quantité de cobalt, de préférence, inférieure à 2 millimoles par mole de méthanol et d'un promoteur halogéné, caractérisé en ce que l'on opère en présence initiale

2

d'au moins un iodure ionique et d'au moins un halogénure covalent, le cation de l'iodure ionique étant choisi dans le groupe constitué par les cations des métaux alcalins, les cations des métaux alcalino-terreux, les cations ammonium, les cations ammonium quaternaires, les cations phosphonium et les cations phosphonium-quaternaires, le rapport I⁻/Co (en Atomes-grammes) étant au moins égal à 5 et le nombre d'atomes-grammes d'halogène, en provenance de l'halogénure covalent mis en œuvre représentant de 0,1 à 10 % du nombre d'ions-grammes I⁻.

Selon l'invention, il a été découvert de manière tout à fait surprenante que des résultats remarquables, exprimés notamment en terme de productivité en acétaldéhyde, peuvent être obtenus par l'utilisation conjointe des deux types de « promoteurs » mentionnés ci-avant, l'iodure ionique étant engagé en quantité telle que le rapport (I⁻/Co) soit supérieur ou égal à 5 et cela avec une très faible proportion d'halogénure covalent.

Le procédé selon l'invention nécessite la mise en œuvre d'au moins un iodure ionique. Par iodure ionique, on entend les iodures minéraux ou organiques pris isolément ou en mélange, dont les cations sont choisis parmi les cations des métaux alcalins, les cations des métaux alcalino-terreux, les cations ammonium, ammonium quaternaires, les cations phosphonium, phosphonium quaternaire. La nature précise du cation ammonium ou phosphonium n'est pas fondamentale, dans le cadre de la présente invention.

Le choix parmi ces composés est davantage orienté par des considérations d'ordre pratique telles que la solubilité, la disponibilité et la commodité d'emploi. A ce titre, les iodures d'ammonium quaternaires dérivés des trialcoylamines inférieures, telles que la triméthylamine, la triéthylamine, la tributylamine, ou bien correspondant soit à des alcoylarylamines telle que la diméthylaniline, soit à des amines tertiaires cycliques telles que les N-alcoyl-pyrrolidines ou pipéridines, les N-alcoylhexaméthylène imines, le pyrrole, la pyridine, les alcoylpyridines, quaternisées par traitement avec des esters de l'acide iodhydrique et d'alcools aliphatiques conviennent particulièrement bien. Les iodures d'ammonium bis-quaternaires, dérivés par exemple des tétraalcoylalcoylène diamines peuvent également être utilisés dans le cadre de la présente invention.

L'utilisation des iodures de phosphonium dérivés par exemple des triarylphosphines, d'accès facile, telles que la triphénylphosphine, la tritolylphosphine, la trixylylphosphine ou des trialkylphosphines d'accès facile telles que la triméthylphosphine, la tributylphosphine ou la tricyclohexylphosphine et quaternisés par des iodures d'alcoyles ou d'aralcoyles, est également envisagée dans le cadre du présent procédé. Les iodures de phosphonium bis-quaternaires, dérivés par exemple des bis-α, ω (diphénylphosphino) alcanes peuvent également être utilisés dans le cadre de la présente invention.

Un mode préféré de réalisation de la présente invention comprend l'utilisation d'iodures des métaux alcalins ou alcalino-terreux, tels que LiI, NaI, KI, CsI et CaI₂. De préférence on utilise un ou plusieurs iodures des métaux alcalins et de manière encore plus avantageuse NaI ou KI.

Selon la présente invention, le rapport (I⁻/Co), I⁻ provenant de l'iodure ionique, doit être au moins égal à 5. Bien que ce rapport puisse varier dans de larges limites, il ne semble pas utile de dépasser une valeur de l'ordre de 200. Le rapport (I⁻/Co) est de préférence supérieur ou égal à 10. Selon un mode de réalisation particulièrement avantageux du procédé selon l'invention, le rapport I⁻/Co est supérieur ou égal à 20.

Le procédé selon l'invention exige également la mise en œuvre d'au moins un halogénure covalent. Par halogénure covalent, on entend d'une part les chlorures, les bromures et, de préférence, les iodures d'hydrocarbyles, c'est-à-dire, des composés formés par remplacement, au moyen d'halogène, d'au moins un atome d'hydrogène d'une molécule organique ne contenant que du carbone et de l'hydrogène, et, d'autre part, les composés qui dans les conditions de réaction sont susceptibles de conduire à l'apparition d'un halogénure de méthyle dans le milieu réactionnel, choisis parmi Cl₂, Br₂, I₂, HCl, HBr, HI, CoBr₂ et CoI₂.

L'invention envisage notamment l'utilisation des chlorures, bromures et iodures d'alcoyles inférieurs ayant de 1 à 4 atomes de carbone dans la molécule, tels que le bromure et l'iodure de méthyle, le bromure et l'iodure d'éthyle.

De préférence, on utilise l'iodure de méthyle ou l'une de ses sources potentielles choisies parmi l'iode, l'acide iodhydrique et l'iodure de Cobalt.

Selon la présente invention, l'effet apporté par l'halogénure covalent est appréciable même pour de faibles teneurs, de l'ordre de 0,1 atome-gramme d'halogène (désigné par X dans ce qui suit) pour 100 ions-grammes d'Iodure, c'est-à-dire pour un rapport X/I⁻ de l'ordre de 0,1 %.

Il n'est pas souhaitable de dépasser la valeur de 10 % pour ce rapport, notamment pour des raisons technologiques, en particulier pour limiter la corrosion de l'appareillage.

On opère de préférence avec un rapport X/I⁻ compris entre 1 et 8 %.

Le procédé selon l'invention est réalisé en présence de cobalt. N'importe quelle source de cobalt qui réagira avec l'oxyde de carbone dans le milieu de réaction pour donner un complexe cobalt-carbonyle, hydrocobalt-carbonyle ou cobalt-carbonylate peut être utilisé dans le cadre de la présente invention.

Des sources typiques de cobalt sont par exemple le cobalt métallique finement divisé, ces sels inorganiques tel que le carbonate de cobalt, des sels organiques, en particulier d'un acide gras. Peuvent également être employés les cobalt-carbonyles, hydrocarbonyles, ou leurs complexes. Parmi les dérivés du cobalt convenant pour la mise en œuvre du procédé selon l'invention, on peut citer l'acétate et le formiate de cobalt, les halogénures de cobalt, en particulier l'iodure de cobalt, le dicobaltoctacarbonyle.

On opère avec une quantité efficace de cobalt. En général, cette quantité est telle que le rapport du nombre de milliatomes-grammes de cobalt au nombre de moles de méthanol ($Co/CH_3/OH$) soit compris entre 0,01 et 1, et de préférence entre 0,05 et 0,4.

Bien entendu lorsque la source de cobalt est un halogénure il n'est pas indispensable d'introduire un halogénure covalent, tel que $X_2$ ou $CH_3X$, X représentant un atome de chlore, de brome ou d'iode. Toutefois l'addition d'un tel composé peut s'avérer avantageuse.

Le procédé de carbonylation selon la présente invention est conduit de préférence en phase liquide. Comme on opère le plus souvent avec du méthanol en excès, l'emploi simultané d'un solvant supplémentaire est généralement superflu mais on peut en principe faire usage de tels solvants, par exemple d'hydrocarbures, d'esters, d'éthers et des produits de la réaction.

Dans le cadre du présent procédé, il n'est pas nécessaire de purifier ou de déshydrater le méthanol au préalable. On peut utiliser du méthanol de qualité technique. Le méthanol utilisé au départ peut contenir jusqu'à 50 % d'eau et/ou d'acétate de méthyle.

De même, on peut utiliser dans le cadre de la présente invention les halogénures covalents, les iodures ioniques et les composés à base de cobalt, du commerce.

Selon le présent procédé, on fait réagir sur le méthanol un mélange de monoxyde de carbone et d'hydrogène. Il est essentiel que ledit mélange renferme au moins 25 % en mole d'hydrogène. En général, on peut utiliser des mélanges renfermant jusqu'à 95 % d'hydrogène. On utilise de préférence des mélanges renfermant de 40 à 80 % d'hydrogène. Le mélange de gaz peut renfermer des impuretés telles que, par exemple, du dioxyde de carbone, de l'oxygène, du méthane et de l'azote.

La réaction nécessite la mise en œuvre d'une pression totale, généralement comprise entre 50 et 600 bars. De préférence, la pression totale est comprise entre 75 et 350 bars, et plus particulièrement entre 100 et 320 bars.

La température de réaction est d'au moins 165 °C environ et peut atteindre 240 °C lorsque l'on opère sans solvant. Lorsque l'on met en œuvre un solvant, ce qui demeure facultatif dans le cadre de la présente invention, la température peut atteindre 300 °C.

De préférence, la température de réaction se situe dans la gamme allant de 180 °C à 230 °C.

Les exemples ci-après illustrent la mise en œuvre du procédé selon l'invention sans toutefois en limiter le domaine ou l'esprit.

Les conventions utilisées dans les exemples ci-après sont les suivantes :

AcH désigne l'acétaldéhyde
AcOMe désigne l'acétate de méthyle
AcOH désigne l'acide acétique
EtOH désigne l'éthanol
$C_4$ désigne l'ensemble des produits formés, ayant 4 atomes de carbone dans la molécule, à savoir le buténal et/ou le butanal et/ou le butanol.

MeOMe désigne le diméthyléther (produit recyclable dans la réaction).

— RR pour un produit donné, est égal au rapport du nombre de moles du produit considéré, formées pendant la réaction, au nombre de moles de méthanol engagées dans la réaction, à l'exception de RR ($C_4$) et de RR (MeOMe) qui sont égaux au double du rapport défini ci-avant.

— $\Sigma RR$ est la somme des RR définis ci-avant, pour l'ensemble des produits, à l'exception du diméthyléther.

N.B. dans l'ensemble des exemples ci-après, RR(EtOH)/$\Sigma RR$ est inférieur à 1 %.

## Essais témoins (a à e)

Une première série d'essais témoins (a, b et c) a été réalisée selon un mode opératoire analogue à celui décrit ci-après pour l'essai témoin b.

Dans un autoclave en acier inoxydable Z 8-CNDT 17-12 (norme AFNOR) de 125 ml de capacité, on charge :

50 ml (1 229 m Mol) de méthanol
0,042 g (0,29 m Mol) d'iodure de méthyle
0,010 g (0,058 m At-g) de dicobalt octacarbonyle

Après fermeture de l'autoclave, on établit une pression de 180 bars à l'aide d'un mélange $CO/H_2$ : 1/1. L'agitation par un système de va-et-vient est mise en route et l'autoclave est porté à 200 °C, en 25 mn environ, au moyen d'un four annulaire. La pression totale dans l'autoclave est alors de 250 bars et elle est maintenue entre 240 et 254 bars par des recharges périodiques du mélange $CO/H_2$. Après 2 heures de réaction à la température indiquée (absorption totale de 56 bars du mélange $CO/H_2$), le chauffage et l'agitation sont arrêtés ; l'autoclave est refroidi et dégazé.

Le mélange réactionnel résultant est analysé par chromatographie gazeuse, après avoir été dilué à l'eau et acidifié par de l'acide sulfurique 36 N.

Les conditions particulières ainsi que les résultats obtenus sont portés dans le tableau I, ci-après.

Une deuxième série d'essais témoins (d et e) a été réalisée selon un mode opératoire analogue à celui décrit ci-après pour l'essai témoin d.

Dans un autoclave en acier inoxydable Z 8-CNDT 17-12 (norme AFNOR) de 250 ml de capacité, on charge :

100 ml (2 437 m Mol) de méthanol

2 g (12 m Mol) d'iodure de potassium

0,020 g (0,117 m At-g) de dicobalt octacarbonyle

Après fermeture de l'autoclave, on établit sur pression de 140 bars à l'aide d'un mélange CO/H$_2$ : 1/1.

L'agitation par un système classique de va-et-vient est mise en route, et l'autoclave est porté à 200 °C au moyen d'un four annulaire. La pression totale dans l'autoclave est alors de 255 bars. Cette pression est maintenue entre 248 et 255 bars par des recharges périodiques du mélange CO/H$_2$.

Après 90 mn de réaction à la température indiquée (absorption totale de 42 bars du mélange CO/H$_2$) le chauffage et l'agitation sont arrêtés. L'autoclave est alors refroidi et dégazé. Le mélange réactionnel résultant est alors analysé comme précédemment.

Les conditions particulières ainsi que les résultats obtenus sont portés dans le tableau I ci-après.

### Tableau I — Essais témoins

| Témoin | Co/CH$_3$OH | Iodure Ionique Nature | I$^-$/Co | Halogénure Covalent Nature | X/Co | ΣRR en % | $\frac{RR}{\Sigma RR}$ (%) | AcH Productivité g/h × 1 | AcH Productivité g/h × Co (*) | MeOMe RR % | $\frac{RR}{\Sigma RR}$ (%) C$_4$ | Sous-produits $\frac{RR}{\Sigma RR}$ (%) AcOH +AcOMe |
|---|---|---|---|---|---|---|---|---|---|---|---|---|
| a (**) | 0,047 | — | 0 | — | 0 | 0 | — | 0 | 0 | 0 | — | — |
| b | 0,047 | — | 0 | CH$_3$I | 5 | 6 | 94 | 30 | 440 | 5.1 | — | 6 |
| c | 0,047 | — | 0 | CH$_3$I | 100 | 9.4 | 94 | 47 | 680 | ≥ 16. | 0 | 6 |
| d | 0,048 | K I | 102 | — | 0 | 4.2 | 92.5 | 28.0 | 405 | 0.7 | 2 | 5.5 |
| e | 0,049 | (1) | 97 | — | 0 | 5.6 | 91. | 36.6 | 531 | 0.9 | — | 9 |

(**) Essai réalisé sous une pression totale de 270 bars.

(1) : [(CH$_3$)(C$_4$H$_9$)$_3$P] I.

(*) Productivité exprimée en gramme d'AcH formé par gramme de cobalt engagé et par heure.

## Exemples 1 à 10

Ces exemples illustrent l'utilisation de divers iodures ioniques.

On utilise un mode opératoire analogue à celui décrit précédemment pour l'essai témoin (d)

Les conditions communes sont les suivantes :

Mélange CO/H$_2$ = 1 : 1

Température de l'ordre de 200 °C

Pression totale : 250 bars

Source de cobalt : Co$_2$(CO)$_8$

Co/CH$_3$OH compris entre 0,045 et 0,05 sauf indications contraires.

Halogénure covalent : CH$_3$I

Les conditions particulières ainsi que les résultats obtenus sont portés dans le Tableau II, ci-après.

### Tableau II — Exemples 1 à 10

| N° Exemple | Iodure Ionique Nature | I$^-$/Co | X/I$^-$ en % | Durée en mn | ΣRR en % | AcH RR/ΣRR % | AcH Productivité g/h × 1 | AcH Productivité g/h × Co (*) | MeOMe RR en % | C$_4$ RR/ΣRR (en %) |
|---|---|---|---|---|---|---|---|---|---|---|
| 1 | NaI | 107 | 4.9 | 90 | 20.8 | 85 | 126 | 1900 | 3.3 | 3. |
| 2 | KI | 102 | 4.8 | 90 | 17.6 | 87 | 109 | 1560 | 3. | 1.6 |
| 3 | LiI | 102 | 4.8 | 90 | 19.7 | 84 | 118 | 1710 | 2.7. | 3.9 |
| 4 | CsI | 102 | 4.8 | 90 | 15.6 | 87 | 97 | 1370 | 3.2 | 1.9 |
| 5 (α) | CaI$_2$ | 93 | 5.9 | 40 | 13.6 | 69 | 150 | 710 | 1.3. | 21.5 |
| 6 | NH$_4$I | 102 | 2.5 | 30 | 4.2 | 84 | 75 | 1100 | 3.6 | — |
| 7 | [(CH$_3$)(nC$_4$H$_9$)$_3$N] I | 97 | 2.5 | 45 | 10.1 | 90 | 130 | 1830 | 1.4 | — |
| 8 | [(CH$_2$)(n−C$_4$H$_9$)$_3$P] I | 99 | 5.0 | 90 | 18.9 | 85 | 114 | 1650 | 4. | 2.0 |
| 9 (β) | [(CH$_3$)(C$_6$H$_{11}$)$_3$P] I | 95 | 2.5 | 45 | 12.1 | 86 | 148 | 2040 | 1.7. | — |
| 10 | [(CH$_3$)(C$_6$H$_5$)$_3$P] I | 95 | 2.5 | 45 | 12.2 | 86 | 150 | 2060 | 1.6. | — |

(α) : essai réalisé avec Co/CH$_3$OH = 0,148.

(β) : C$_6$H$_{11}$ désigne un radical cyclohexyl.

(*) : même remarque que dans le Tableau I.

# 0 011 042

## Exemples 11 à 13

Ces exemples illustrent l'utilisation de divers promoteurs halogénés.

On opère comme dans l'essai témoin (b), décrit ci-avant.

Les conditions communes sont les suivantes :

Mélange $CO/H_2$ à 50 % (molaire) d'$H_2$

Température : 200 °C

Pression totale : 250 bars

Durée de la réaction : 40 mn

Source de cobalt : $Co_2(CO)_8$, $Co/CH_3OH = 0,144$

Iodure ionique : KI, $I^-/Co = 34$

Halogénure covalent : $X/I^- = 5,8$ %

Les conditions particulières ainsi que les résultats obtenus sont portés dans le Tableau III, ci-après :

### Tableau III — Exemples 11 à 13

| Exemple | Halogénure Covalent | $\Sigma RR$ en % | $\frac{RR}{\Sigma RR}$ en % | AcH Productivité g/h × 1 | AcH Productivité g/h × 1 (*) | MeOMe RR en % |
|---|---|---|---|---|---|---|
| 11 | $C_2H_5Br$ | 15,1 | 91 | 225 | 1090 | 1,3 |
| 12 | $C_2H_5I$ | 15,8 | 91 | 230 | 1110 | 1,2 |
| 13 | $CH_3I$ | 16,3 | 90 | 240 | 1160 | 1,4 |

NB : on ne voit pas apparaître de $C_4$ dans ces essais.

(*) Cf. Tableaux précédents.

## Exemples 14 à 17

Les exemples ci-après illustrent la mise en œuvre de diverses pressions totales.

On opère comme dans l'essai témoin (b).

Les conditions communes sont les suivantes :

Température = 200 °C

Durée de réaction = 40 mn

Source de Cobalt : $Co_2(CO)_8$, $Co/CH_3OH = 0,047$

Iodure ionique : KI, $I^-/Co = 207$

Halogénure covalent : $CH_3I$, $X/I^- = 2,8$ %

Les conditions particulières ainsi que les résultats obtenus sont portés sur le Tableau IV ci-après :

### Tableau IV — Exemples 14 à 17

| Exemple | Pression Totale (Bars) | $H_2$ en % Molaire | $\Sigma RR$ (%) | $\frac{RR}{\Sigma RR}$ (%) | AcH Productivité g/l × h | AcH Productivité g/h × Co (*) | MeOMe RR % | $C_4$ $\frac{RR}{\Sigma RR}$ (%) |
|---|---|---|---|---|---|---|---|---|
| 14 | 300 | 44,5 | 10 | 89 | 144 | 2080 | 1,6 | — |
| 15 | 250 | 47 | 9,2 | 89 | 130 | 1890 | 1,6 | — |
| 16 | 225 | 54 | 7,3 | 92 | 109 | 1580 | 1,6 | — |
| 17 | 300 | 56 | 9,6 | 93 | 146 | 2100 | 1,8 | — |

NB : On n'observe pas de $C_4$ dans cette série d'essais.

(*) Cf. Tableaux précédents.

## Exemple 18

En reproduisant le mode opératoire de l'essai témoin (d) on a chargé 1,22 moles de méthanol, 50 ml de tétraglyme, 0,35 milliatome-gramme de cobalt sous la forme de $CoI_2$, 12 millimoles de KI et 0,70 millimole de $CH_3I$. La température de réaction, atteinte en 20 minutes, est de 200 °C, pour une pression totale de 250 bars, la teneur en hydrogène du mélange gazeux étant de 50 % (molaire). En 90 minutes de

6

réaction on a obtenu de 390 g d'acétaldéhyde par heure et par gramme de cobalt, avec une sélectivité (RR/ΣRR) de 83 %.

ΣRR est égal à 27,4 % et on observe peu de $C_4$ (RR/ΣRR = 6 %).

## Exemple 19

En reproduisant le mode opératoire de l'essai témoin (d) on a chargé 2,44 moles de méthanol, 0,35 milliatome-gramme de cobalt sous la forme de $Co_2(CO)_8$, 12 millimoles de KI et 0,70 millimole de $CH_3I$. La température de réaction, atteinte en 20 mn, est de 200 °C, pour une pression totale de 250 bars, la teneur en hydrogène du mélange gazeux étant de 50 % (molaire). En 40 mn de réaction on a obtenu 1 280 g d'acétaldéhyde par heure et par gramme de cobalt, avec une sélectivité de 87 %.

ΣRR est égal à 19 % et on n'observe pratiquement pas de $C_4$ (RR/ΣRR ≤ 1 %).

## Exemple 20

En reproduisant le mode opératoire de l'essai témoin (d) on a chargé 2,44 moles de méthanol, 0,181 milliatome-gramme de cobalt sous la forme de $Co_2(CO)_8$, 12 millimoles de KI et 0,7 millimole de $CH_3I$. La température de réaction, atteinte en 20 mn, est de 200 °C, pour une pression totale de 260 bars, la teneur en hydrogène du mélange gazeux étant de 60 puis 50 % (molaire). En 40 mn de réaction on a obtenu 2 090 g d'acétaldéhyde par heure et par gramme de cobalt, avec une sélectivité de 91,5 %.

ΣRR est égal à 15,2 % et on n'observe pratiquement pas de $C_4$.

## Exemple 21

En reproduisant le mode opératoire de l'essai témoin (d) on a chargé 2,44 moles de méthanol, 0,118 milliatome-gramme de cobalt sous la forme de $Co_2(CO)_8$, 12 millimoles de KI et 0,23 millimole de $CH_3I$. La température de réaction, atteinte en 20 mn, est de 200 °C, pour une pression totale de 250 bars, la teneur en hydrogène du mélange gazeux étant de 50 % (molaire). En 90 minutes de réaction on a obtenu 1 350 g d'acétaldéhyde par heure et par gramme de cobalt, avec une sélectivité de 91 %.

ΣRR est égal à 14,5 % et on n'observe pratiquement pas de $C_4$.

## Exemple 22

En reproduisant le mode opératoire de l'essai témoin (d) on a chargé 2,44 moles de méthanol, 0,35 milliatome-gramme de cobalt sous la forme de $CoI_2$, 11,3 millimoles de KI et 0,7 millimole de $CH_3I$. La température de réaction, atteinte en 20 minutes, est de 200 °C, pour une pression totale de 265 bars, la teneur en hydrogène du mélange gazeux étant de 50 % molaire. En 40 mn de réaction on a obtenu 1 260 g d'acétaldéhyde par heure et par gramme de cobalt, avec une sélectivité de 87 %.

ΣRR est égal à 18,6 % et on n'observe pratiquement pas de $C_4$.

## Exemple 23

En reproduisant le mode opératoire de l'essai témoin (d) on a chargé 2,44 moles de méthanol, 0,136 milliatome-gramme de cobalt sous la forme de $Co_2(CO)_8$, 12 millimoles de KI et 0,70 millimoles de $CH_3I$. La température de réaction, atteinte en 20 minutes, est de 200 °C, pour une pression totale de 200 bars, la teneur en hydrogène du mélange gazeux étant de 45 % puis de 50 % (molaire). En 40 mn de réaction, on a obtenu 1 980 g d'acétaldéhyde par heure et par gramme de cobalt, avec une sélectivité de 91 %.

ΣRR est égal à 10,9 % et on n'observe pratiquement pas de $C_4$.

## Exemple 24

En reproduisant le mode opératoire de l'essai témoin (b) on a chargé 1,233 moles de méthanol, 0,117 milliatome-gramme de cobalt sous la forme de $Co_2(CO)_8$, 12 millimoles de KI et 0,23 millimole de $CH_3I$. La température de réaction, atteinte en 20 minutes, est de 200 °C, pour une pression totale de 250 bars, la teneur en hydrogène du mélange gazeux étant de 50 % molaire. En 40 mn de réaction on a obtenu 1 300 g d'acétaldéhyde par heure et par gramme de cobalt, avec une sélectivité de 89,5 %.

ΣRR est égal à 12,3 % et on n'observe pratiquement pas de $C_4$.

## Exemple 25

En reproduisant le mode opératoire de l'essai témoin (b) on a chargé 1,25 moles de méthanol, 0,175 milliatome-gramme de cobalt sous forme de $Co_2(CO)_8$, 6 millimoles de KI et 0,34 millimoles de $CH_3I$. La température de réaction, atteinte en 20 minutes, est de 160 °C, pour une pression totale de 250 bars, la teneur en hydrogène du mélange gazeux étant de 50 % (molaire). En 40 minutes de réaction on a obtenu 450 g d'acétaldéhyde par heure et par gramme de cobalt, avec une sélectivité de 83 %.

Exemple 26

En reproduisant le mode opératoire de l'essai témoin (d) on a chargé 2,44 moles de méthanol, 0,38 milliatome-gramme de cobalt sous la forme de $CoCO_3$, 12 millimoles de KI et 0,7 millimole de $CH_3I$. La température de réaction, atteinte en 20 minutes, est de 200 °C, pour une pression totale de 250 bars, la teneur en hydrogène du mélange gazeux étant de 50 % molaire. En 40 mn de réaction on a obtenu 1 100 g d'acétaldéhyde par heure et par gramme de cobalt, avec une sélectivité de 88 %.

$\Sigma RR$ est égal à 16,7 % et on n'observe pratiquement pas de $C_4$.

Exemple 27

En reproduisant le mode opératoire de l'essai témoin (d) on a chargé 2,44 moles de méthanol, 0,117 milliatome-gramme de cobalt sous la forme de $Co_2(CO)_8$, 11,6 millimoles de $[(CH_3)(n\text{-}C_4H_9)_3P]$ I et 0,116 millimole de $CH_3I$. La température de réaction, atteinte en 20 minutes, est de 200 °C, pour une pression totale de 250 bars, la teneur en hydrogène du mélange gazeux étant de 50 % (molaire). En 90 minutes de réaction, on a obtenu 880 g d'acétaldéhyde par heure et par gramme de cobalt, avec une sélectivité de 91 %.

$\Sigma RR$ est égal à 9,4 % et on n'observe pratiquement pas de $C_4$.

Exemple 28

En reproduisant le mode opératoire de l'essai témoin (b) on a chargé 1,24 moles de méthanol, 0,175 milliatome-gramme de cobalt sous la forme de $Co_2(CO)_8$, 6 millimoles de KI et 0,34 millimole de $CH_3I$. La température de réaction atteinte en 20 minutes, est de 180 °C, pour une pression totale de 250 bars, la teneur en hydrogène du mélange gazeux étant de 50 % molaire. En 40 mn de réaction, on a obtenu 860 g d'acétaldéhyde par heure et par gramme de cobalt, avec une sélectivité de 87 %.

$\Sigma RR$ est égal à 12,7 % et on n'observe pratiquement pas de $C_4$.

**Revendications**

1. Procédé de préparation de l'acétaldéhyde par réaction sur le méthanol d'un mélange comprenant de l'hydrogène et du monoxyde de carbone, l'hydrogène représentant au moins 25 % en mole, à une température de l'ordre de 165 °C au moins, en présence d'une quantité de cobalt, de préférence, inférieure à 2 millimoles par mole de méthanol et d'un promoteur halogéné, caractérisé en ce que l'on opère en présence initiale d'au moins un iodure ionique et d'au moins un halogénure covalent, le cation de l'iodure ionique étant choisi dans le groupe constitué par les cations des métaux alcalins, les cations des métaux alcalino-terreux, les cations ammonium, les cations ammonium quaternaires, les cations phosphonium et les cations phosphonium quaternaires, l'halogénure covalent étant choisi parmi les chlorures, bromures et iodures d'hydrocarbyles, le chlore, le brome et l'iode moléculaires, les acides halohydriques correspondants, le bromure et l'iodure de cobalt, le rapport $I^-/Co$ (en Atomes-grammes) étant supérieur ou égal à 5, et le rapport $X/I^-$ (en Atomes-grammes) dans lequel X représente un atome d'halogène en provenance d'un halogénure covalent étant compris entre 0,1 et 10 %.

2. Procédé selon la revendication 1, caractérisé en ce qu'on opère en phase liquide.

3. Procédé selon la revendication 1, caractérisé en ce que $X/I^-$ est compris entre 1 et 8 %.

4. Procédé selon les revendications 1 ou 3, caractérisé en ce que l'iodure ionique est choisi dans le groupe constitué par les iodures alcalins et les iodures alcalino-terreux.

5. Procédé selon la revendication 4, caractérisé en ce que l'iodure ionique est choisi dans le groupe constitué par LiI, NaI, KI, CsI et $CaI_2$.

6. Procédé selon les revendications 1 ou 5, caractérisé en ce que $I^-/Co$ est supérieur ou égal à 10.

7. Procédé selon la revendication 6, caractérisé en ce que $I^-/Co$ est compris entre 20 et 200.

8. Procédé selon les revendications 1 ou 3, caractérisé en ce que l'halogénure covalent est choisi dans le groupe constitué par les chlorures, les bromures et les iodures d'alcoyles ayant de 1 à 4 atomes de carbone dans la molécule, le chlore, le brome et l'iode moléculaires, les acides halohydriques correspondants, le bromure et l'iodure de cobalt.

9. Procédé selon la revendication 8, caractérisé en ce que l'halogénure covalent est choisi parmi l'iodure de méthyle, l'iode moléculaire et l'acide iodhydrique.

10. Procédé selon la revendication 1, caractérisé en ce que la quantité de cobalt présente dans le milieu est comprise entre 0,01 et 1 milliatome-gramme par mole de méthanol.

11. Procédé selon la revendication 1, caractérisé en ce que la température de la réaction est comprise entre 180 et 230 °C.

12. Procédé selon la revendication 1, caractérisé en ce que la pression totale est comprise entre 50 et 600 bars, et de préférence entre 75 et 350 bars.

13. Procédé de préparation de l'acétaldéhyde par réaction en phase liquide, sur le méthanol, d'un mélange comprenant de l'hydrogène et du monoxyde de carbone, l'hydrogène représentant au moins 25 % en mole, à une température comprise entre 180 et 230 °C, sous une pression totale comprise entre 75 et 350 bars, en présence de 0,01 à 1 milliatome-gramme de cobalt par mole de méthanol, en présence initiale d'un iodure alcalin, I⁻/Co étant compris entre 20 et 200, et en présence initiale d'iodure de méthyle, CH₃I/I⁻ étant compris entre 1 et 8 % (molaire).

14. Procédé selon les revendications 4 ou 13, caractérisé en ce que l'iodure ionique est choisi dans le groupe constitué par NaI et KI.

15. Procédé selon les revendications 10 ou 13, caractérisé en ce que la quantité de cobalt est comprise entre 0,05 et 0,3 milliatome-gramme par mole de méthanol.

16. Procédé selon les revendications 12 ou 13, caractérisé en ce que la pression totale est comprise entre 100 et 320 bars.

17. Procédé selon les revendications 1 ou 13, caractérisé en ce que la proportion d'hydrogène est comprise entre 40 et 80 % molaire.

## Claims

1. Process for the preparation of acetaldehyde by reacting, with methanol, a mixture comprising hydrogen and carbon monoxide, the hydrogen representing a least 25 mol %, at a temperature of the order of at least 165 °C, in the presence of an effective amount of cobalt, which is, preferably, less than 2 millimols per mol of methanol, characterised in that the process is carried out in the initial presence of at least one ionic iodide and of at least one covalent halide, the cation of the ionic iodide being chosen from the group consisting of alkali metal cations, alkaline earth metal cations, ammonium cations, quaternary ammonium cations, phosphonium cations and quaternary phosphonium cations, the covalent halide being chosen from the group consisting of the chlorides, bromides and the iodides of hydrocarbyls, molecular chlorine, bromine and iodine and the corresponding hydrogen halide acids, cobalt bromide and iodide, the ratio I⁻/Co (in gram-atoms) being greater than or equal to 5, and the ratio X/I⁻ (in gram-atoms), in which X represents a halogen atom originating from a halogen-containing promoter, being between 0,001 and 0,1.

2. Process according to claim 1, characterised in that it is carried out in the liquid phase.

3. Process according to claim 1, characterised in that X/I⁻ is between 0.01 and 0.08.

4. Process according to claims 1 or 3, characterised in that the ionic iodide is chosen from the group consisting of alkali metal iodides and alkaline earth metal iodides.

5. Process according to claim 4, characterised in that the ionic iodide is chosen from the group consisting of LiI, NaI, KI, CsI, and CaI₂.

6. Process according to claims 1 or 5, characterised in that I⁻/Co is greated than or equal to 10.

7. Process according to claim 6, characterised in that I⁻/Co is between 20 and 200.

8. Process according to claims 1 or 3, characterised in that the covalent halide is chosen from the group consisting of the chlorides, the bromides and the iodides of alkyls having from 1 to 4 carbon atoms in the molecule, molecular chlorine, bromine and iodine, the corresponding hydrogen halide acids, and cobalt bromide and iodide.

9. Process according to claim 8, characterised in that the covalent halide is chosen from the group consisting of methyl iodide, molecular iodine and hydroiodic acid.

10. Process according to claim 1, characterised in that the amount of cobalt present in the medium is between 0.01 and 1 milligram-atom per mol of methanol.

11. Process according to claim 1, characterised in that the reaction temperature is between 180 and 230 °C.

12. Process according to claim 1, characterised in that the total pressure is between 50 and 600 bars and preferably between 75 and 350 bars.

13. Process for the preparation of acetaldehyde by reacting, with methanol, in the liquid phase, a mixture comprising hydrogen and carbon monoxide, the hydrogen representing at least 25 mol %, at a temperature of between 180 and 230 °C, under a total pressure of between 75 and 350 bars, in the presence of 0.01 to 1 milligram-atom of cobalt per mol of methanol, in the initial presence of an alkali metal iodide, I⁻/Co being between 20 and 200, and in the initial presence of methyl iodide, CH₃I/I⁻ being between 0.01 and 0.08 (in mols).

14. Process according to claims 4 or 13, characterised in that the ionic iodide is chosen from the group comprising NaI and KI.

15. Process according to claims 10 or 13, characterised in that the amount of cobalt is between 0.05 and 0.3 milligram-atom per mol of methanol.

16. Process according to claims 12 or 13, characterised in that the total pressure is between 100 and 320 bars.

17. Process according to claims 1 or 13, characterised in that the proportion of hydrogen is between 40 and 80 mol %.

## Patentansprüche

1. Verfahren zur Herstellung von Acetaldehyd durch Einwirkenlassen eines Gemisches aus Wasserstoff und Kohlenmonoxid, das mindestens 25 Mol-% Wasserstoff enthält, auf Methanol bei einer Temperatur im Bereich von mindestens 165 °C, in Gegenwart einer Menge Kobalt, die vorzugsweise weniger als 2 mMol je Mol Methanol beträgt, sowie eines halogenierten Promotors, dadurch gekennzeichnet, daß man in ursprünglicher Gegenwart mindestens eines ionischen Jodids und mindestens eines Kovalenten Halogenids arbeitet, wobei das Kation des ionischen Jodids aus der Gruppe der Alkalimetallkationen, Erdalkalimetallkationen, Ammoniumkationen, quaternäre Ammoniumkationen, Phosphoniumkationen und quaternäre Phosphoniumkationen augewählt ist, wobei das kovalente Halogenid ausgewählt wird aus der Gruppe bestehend aus den Kohlenwasserstoffchloriden, -bromiden und- jodiden, molekularem Chlor, Brom and Iod, den entsprechenden Halogenwasserstoffsaüren, Kobaltbromid und Kobaltjodid, das Verhältnis J$^-$/Co (in gAtom) gleich oder größer 5 ist und das Verhältnis X/J$^-$ (in gAtom), wobei X für ein Halogenatom steht und aus einem Kovalenten Halogenid stammt, 0,1 bis 10 % beträgt.

2. Verfahren nach Anspruch 1, dadurch gekennzeichnet, daß man in flüssiger Phase arbeitet.

3. Verfahren nach Anspruch 1, dadurch gekennzeichnet, daß X/J$^-$ 1 bis 8 % beträgt.

4. Verfahren nach Anspruch 1 oder 3, dadurch gekennzeichnet, daß das ionische Jodid ausgewählt wird aus der Gruppe, bestehend aus den Alkalijodiden und Erdalkalijodiden.

5. Verfahren nach Anspruch 1 oder 3, dadurch gekennzeichnet, daß das ionische Jodid ausgewählt wird aus der Gruppe, bestehend aus LiJ, NaJ, KJ, CsJ und CaJ$_2$.

6. Verfahren nach Anspruch 1 oder 5, dadurch gekennzeichnet, daß J$^-$/Co größer oder gleich 10 ist.

7. Verfahren nach Anspruch 6, dadurch gekennzeichnet, daß J$^-$/Co 20 bis 200 beträgt.

8. Verfahren nach Anspruch 1 oder 3, dadurch gekennzeichnet, daß das kovalente Halogenid ausgewählt wird aus der Gruppe bestehend aus den Alkylchloriden, Alkylbromiden und Alkyljodiden mit jeweils 1 bis 4 Kohlenstoffatomen im Molekül, molekularem Chlor, Brom oder Jod, den entsprechenden Halogenwasserstoffsaüren, Kobaltbromid und Kobaltjodid.

9. Verfahren nach Anspruch 8, dadurch gekennzeichnet, daß das kovalente Halogenid ausgewählt wird aus der Gruppe bestehend aus Methyljodid, molekularem Jod und Jodwasserstoffsäure.

10. Verfahren nach Anspruch 1, dadurch gekennzeichnet, daß die im Medium vorhandene Menge Kobalt 0,01 bis 10 mgAtom je Mol Methanol beträgt.

11. Verfahren nach Anspruch 1, dadurch gekennzeichnet, daß die Reaktionstemperatur 180 bis 230 °C beträgt.

12. Verfahren nach Anspruch 1, dadurch gekennzeichnet, daß der Gesamtdruck 50 bis 600 bar, vorzugsweise 75 bis 350 bar beträgt.

13. Verfahren zur Herstellung von Acetaldehyd mittels Umsetzung in flüssiger Phase von Methanol mit einem Gemisch, das Wasserstoff und Kohlenmonoxid enthält, wobei der Wasserstoff mindestens 25 Mol-% ausmacht, bei einer Temperatur von 180 bis 230 °C, unter einem Gesamtdruck von 75 bis 350 bar, in Gegenwart von 0,01 bis 1 mgAtom Kobalt je Mol Methanol, in ursprünglicher Gegenwart eines Alkalijodids, wobei J$^-$/Co 20 bis 200 beträgt, sowie in ursprünglicher Gegenwart von Methyljodid, wobei CH$_3$J/J$^-$ 1 bis 8 Mol-% beträgt.

14. Verfahren nach Anspruch 4 oder 13, dadurch gekennzeichnet, daß als ionisches Jodid NaJ oder KJ gewählt wird.

15. Verfahren nach Anspruch 10 oder 13, dadurch gekennzeichnet, daß die Menge Kobalt 0,05 bis 0,3 mgAtom je Mol Methanol beträgt.

16. Verfahren nach Anspruch 12 oder 13, dadurch gekennzeichnet, daß der Gesamtdruck 100 bis 320 bar beträgt.

17. Verfahren nach Anspruch 1 oder 13, dadurch gekennzeichnet, daß der Anteil Wasserstoff 40 bis 80 Mol-% ausmacht.